# EUROPEAN PATENT SPECIFICATION

(11) **EP 0 728 830 B1**
(45) Date of publication and mention of the grant of the patent: **03.05.2000**
(21) Application number: 96102671.3
(22) Date of filing: 22.02.1996
(51) Int. Cl.: C09K 19/46, C09K 19/30

(54) **Liquid crystal composition and liquid crystal display device**
Flüssigkristallzusammensetzung und Flüssigkristallanzeigevorrichtung
Composition liquide cristalline et dispositif d'affichage à cristaux liquides

(30) Priority: 22.02.1995 JP 5982295; 12.05.1995 JP 13862595
(43) Date of publication of application: 28.08.1996
(73) Proprietor: Chisso Corporation, Osaka-shi Osaka 530-0055 (JP)
(72) Inventor: Kondo, Tomoyuki, Ichihara-shi, Chiba-ken (JP); Matsui, Shuichi, Ichihara-shi, Chiba-ken (JP); Koizumi, Yasuyuki, Ichihara-shi, Chiba-ken (JP); Shibata, Koichi, Ichihara-shi, Chiba-ken (JP); Haseba, Yasuhiro, Ichihara-shi, Chiba-ken (JP); Hachiya, Norihisa, Ichihara-shi, Chiba-ken (JP); Nakagawa, Etsuo, Ichihara-shi, Chiba-ken (JP); Miyazawa, Kazutoshi, Ichihara-shi, Chiba-ken (JP)
(74) Representative: Kindler, Matthias, Dr. Dipl.-Chem.

(56) References cited:
- WO-A-92/14800
- WO-A-94/03558
- GB-A- 2 253 402
- GB-A- 2 266 714

## Description

### 1. Field of the Invention

The present invention relates to a novel nematic liquid crystal composition. More specifically, the present invention relates to a liquid crystal composition for active matrix LCD and a liquid crystal display device composed by using the liquid crystal composition.

### 2. Description of the Prior Art

Liquid crystal display devices have been obtained by filling a liquid crystal composition in a sealed cell formed between two substrates provided with transparent electrodes. LCD are low in consumptive electric power compared to CRT (cathode-ray tube display). In addition, LCD can be miniaturized and lightened. Thus, the LCD have been used in practice in such modes as twist nematic (TN) mode, super twisted nematic (STN) mode, and thin film transistor (TFT) scheme. Among them, active matrix LCD (AM-LCD) such as thin film transistor (TFT) has particularly been paid attention as a favorite of a flat display in harmony with the progress toward actualization of color-display devices and highly precision devices.

Liquid crystal compositions for AM-LCD are required to have the following characteristics:
1) They can be provided with a suitable optical anisotropy ( Δn) depending on the thickness of a cell.
2) They have a high voltage holding ratio (VHR) to maintain the contrast of LCD at a high level.
3) They can be provided with a suitable threshold voltage (Vₜₕ) depending on driving circuit.
4) They have a wide mesomorphic range depending on the environment for use.

That is, AM-LCD has adopted, as driving mode, a TN display mode in which molecular alignment of liquid crystal filled between an upper and lower substrates are twisted by 90°. In this TN display mode, coloring due to a interference of a liquid crystal cell caused at the time when voltage is not applied is a problem. In order to avoid this problem and to obtain a suitable contrast, it is necessary to set a product Δn·d of Δn and cell thickness (µm) at a constant value, for example, 0.5 µm. Since there exists such restriction, a main stream of Δn of liquid crystal compositions for TFT which have been practically used has generally become about 0.07 to 0.11, particularly 0.08 to 0.10 for 1st.Min. system.

Further, as seen in the appearance of small size and lightweight notebook type personal computers, development of LCD with an object for portable type is active in recent years. While there are many restrictions in the aspect of driving electric source in respect of such portable LCD, reduction of production cost is desired in addition to further lightening and miniaturizing. As one of the means to answer such requirements, liquid crystal materials of small consumptive electric power that is liquid crystal materials having a low Vth are considered and their development is desired.

Further, accompanied with the development of portable LCD, development of LCD on the assumption of outdoor usage began to be studied. In order that liquid crystal compositions can stand to outdoor use, it is necessary for LCD to display a nematic phase even in a range which exceeds a temperature range under environment for use. From such viewpoint, liquid crystal compositions having a nematic-isotropic phase transition temperature (clearing point: T_{NI}) of 60°C or higher and having a lower limit value of nematic phase transition temperature (T_{L}) of -20°C or lower have become a main stream of liquid crystal compositions for TFT currently being used.

In order to meet such requirements, several kinds of liquid crystalline compounds and liquid crystal compositions comprising the compounds have been developed up to now. For example, a composition having a comparatively large dielectric anisotropy ( Δε) and comprising 15 % by weight of a trifluoro-compounds and 85 % by weight of a difluoro-compound is disclosed in Application Example 2 of Unexamined Japanese Patent Publication No. 2-233626. However, this composition has a defects that its Vₜₕ is high, compatibility of the components comprised -therein is deteriorated particularly at low temperatures, and further, its range of nematic phase is small.

Besides, an example of a composition comprising a trifluoro-compound and a difluoro-compound is disclosed in WO 94/03558. However, the compositions disclosed in its Examples 1 and 2 are insufficient for practical use since they have such a low clearing point as lower than 50°C and have a Δn of lower than 0.06.

Whereas liquid crystal compositions are being earnestly studied according to several purposes as mentioned above, it is not yet sufficient, and it is the present situation that new improvements are continually required.

### SUMMARY OF THE INVENTION

An object of the present invention is to solve the problems of conventional technology as mentioned above and to provide a liquid crystal composition which particularly has a low Vₜₕ, is excellent in compatibility at low temperatures, and has a large nematic phase range while satisfying several characteristics required to liquid crystal compositions for AM-LCD.

As a result of diligent research by the present inventors on compositions which have used several liquid crystalline compounds to achieve the objects mentioned above, it has reached the present invention.

Liquid crystal composition of the present invention is characterized by comprising as the first component, at least one compound expressed by any one of the following formulas (11) to (1-3) 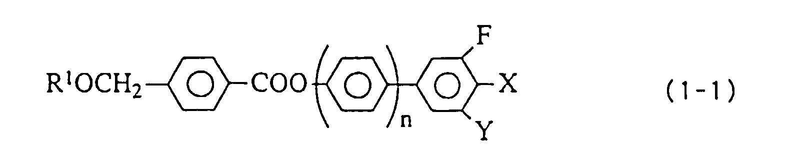 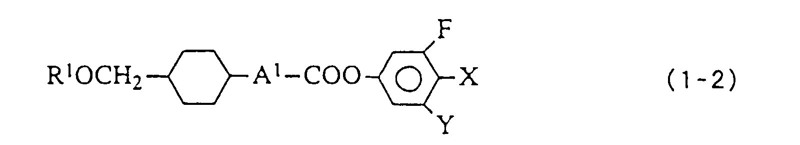 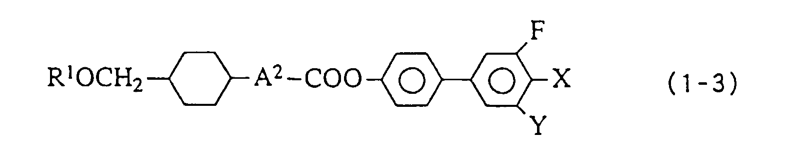 wherein R¹ represents an alkyl group having 1 to 10 carbon atoms, A¹ and A² independently represent trans-1,4-cyclohexylene or 1,4-phenylene, respectively, X represents OCF₃ or CF₃, Y represents H or F, and n represents 0 or 1,
and comprising, as the second component, at least one compound expressed by any one of the following formulas (2-1) to (2-7) 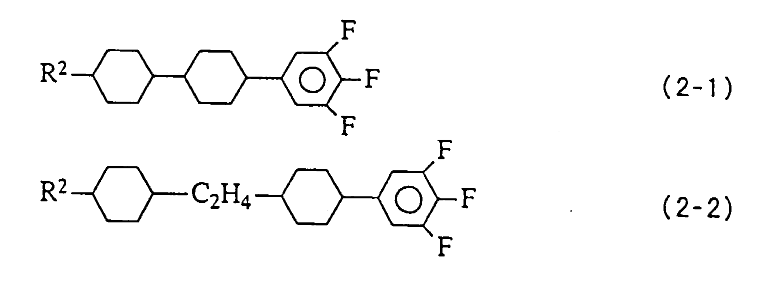 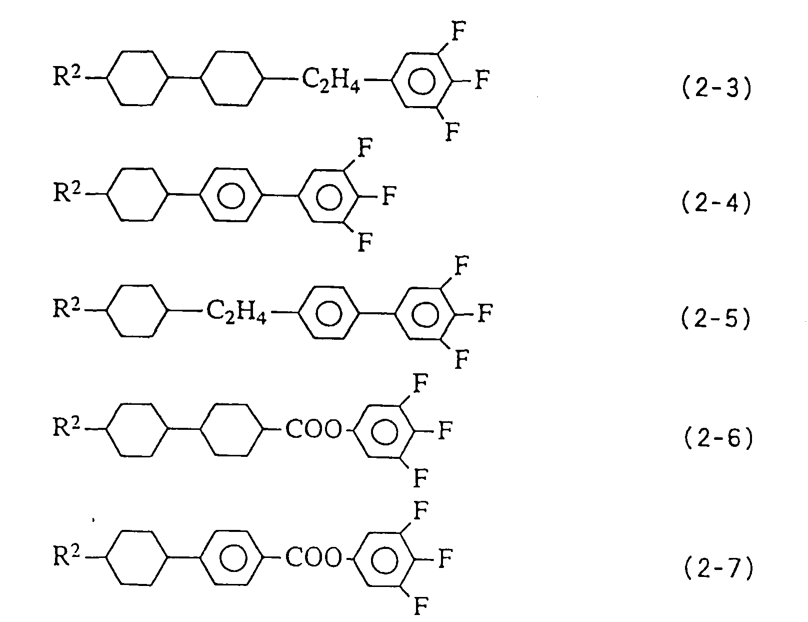 wherein R² represents an alkyl group having 1 to 10 carbon atoms.

In the liquid crystal composition of the present invention mentioned above, the content of the first component and that of the second component are preferably 3 to 40 % by weight (first component) and 60 to 97 % by weight (second component), respectively, based on the total weight of the liquid crystal composition.

Liquid crystal composition of the present invention may additionally comprise at least one compound selected from the group consisting of the first group of compounds expressed by the following formula (3), the second group of compounds expressed by the formula (4-1) and/or (4-2), and the third group of compounds expressed by the formulas (5-1) and/or (5-2). First group compounds: 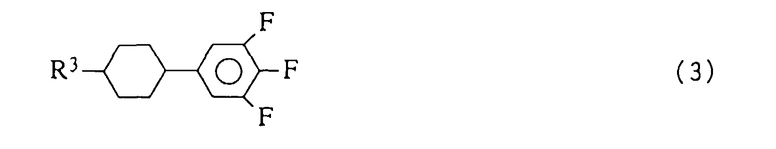 wherein R³ represents an alkyl group having 1 to 10 carbon atoms,
Second group compounds: 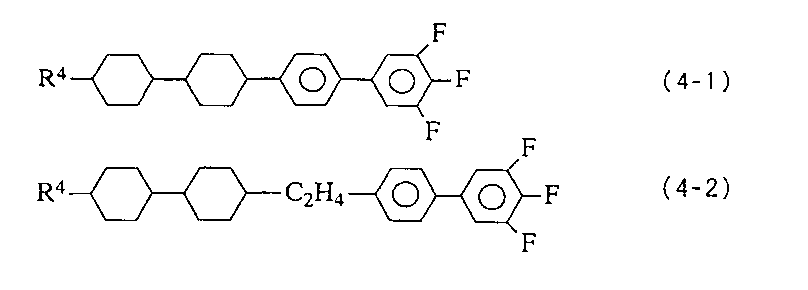 wherein R⁴ represents an alkyl group having 1 to 10 carbon atoms,
Third group compounds: 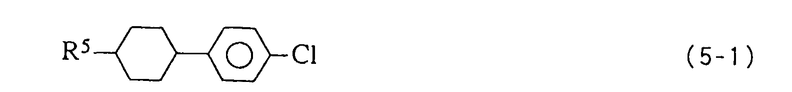 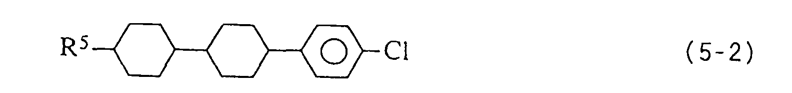 wherein R⁵ represents an alkyl group having 1 to 10 carbon atoms.

By using these liquid crystal compositions of the present invention, liquid crystal display devices which satisfy the objects of the present invention can be obtained.

As preferable examples of the first component used for the liquid crystal compositions of the present invention, the compounds expressed by any one of the formulas (1-1-1) to (1-1-8) for the formula (1-1); the compounds expressed by any one of the formals (1-2-1) to (1-2-8) for the formula (1-2); and the compounds expressed by any one of the formulas (1-3-1) to (1-3-8) for the formula (1-3) can be mentioned. 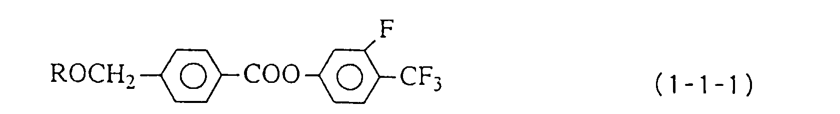 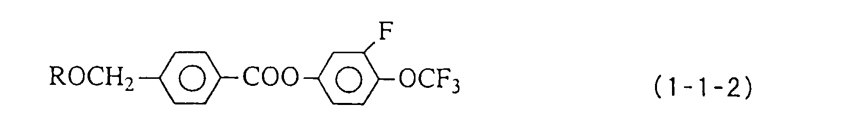 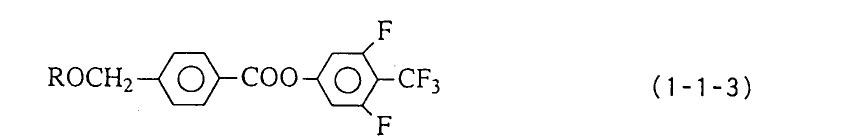 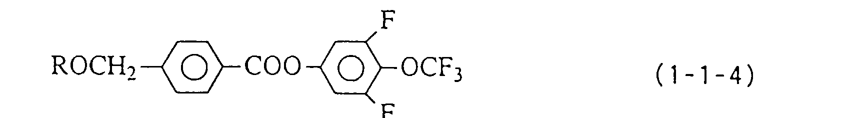 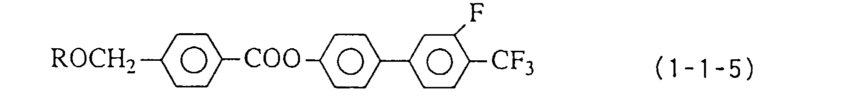 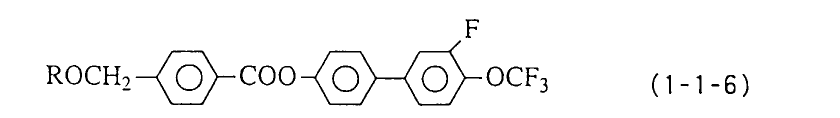 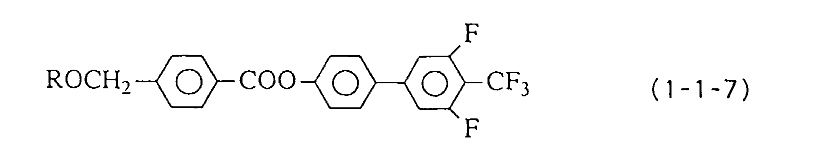 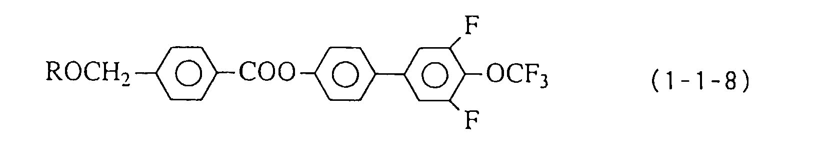 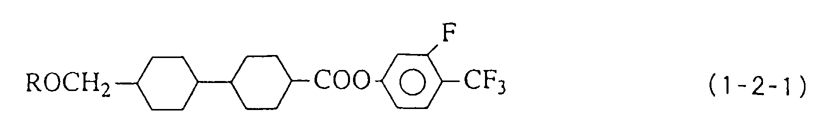 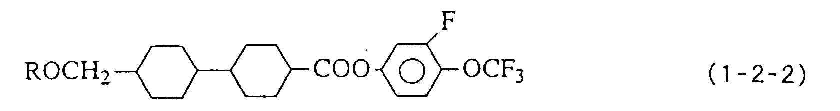 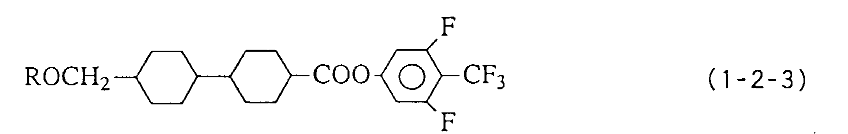 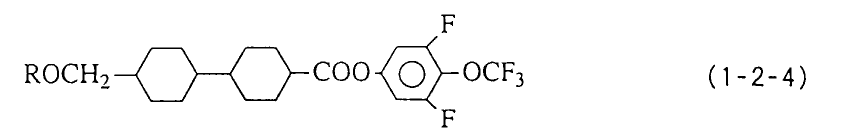 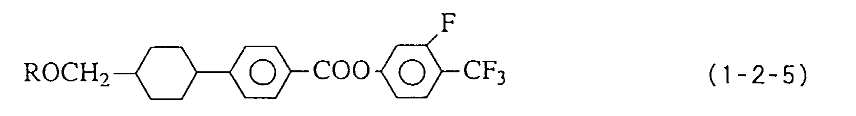 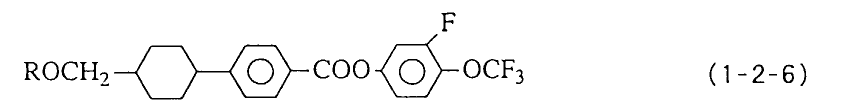 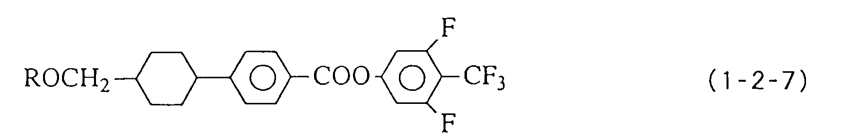 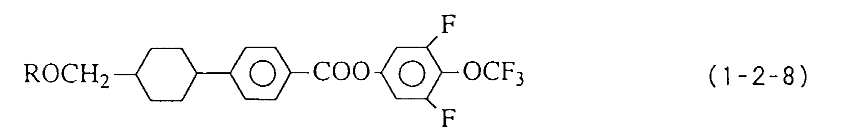 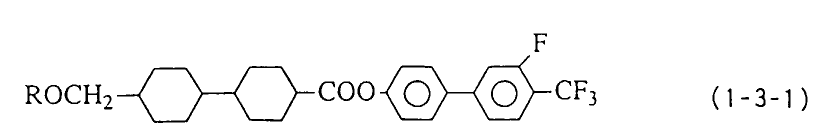 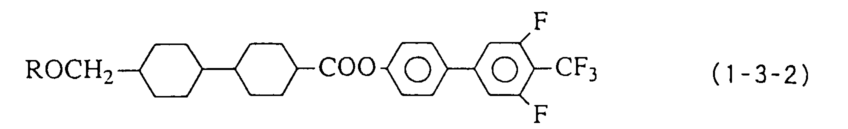 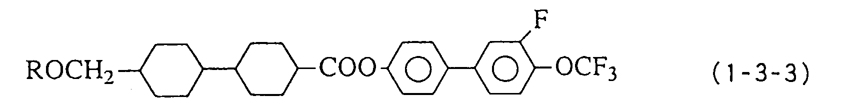 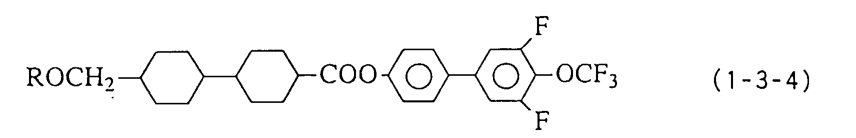 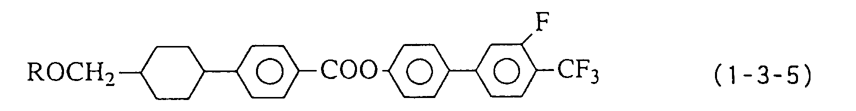 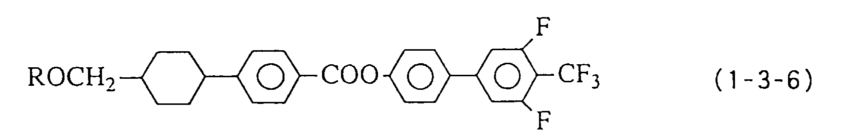 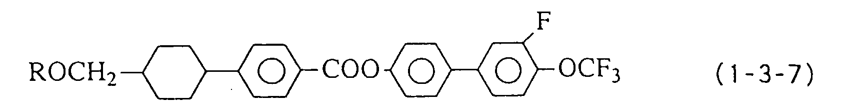 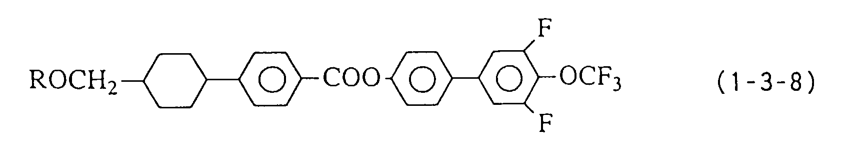 in each of the formulas mentioned above, R represents an alkyl group having 1 to 10 carbon atoms.

Among these compounds, particularly the compounds expressed by any one of the formulas (1-1-2), (1-1-7), (1-2-2), (1-2-3), (1-2-6), (1-2-8), (1-3-2), and (1-3-6) are preferably used.

Since the compounds of the first component generally have a Δε in a range of 10 to 35 and are also excellent in heat stability and chemical stability, they assume the important role of particularly lowering Vₜₕ of liquid crystal compositions for TFT. Especially, since the compounds expressed by the formula (1-3) are tetracyclic compounds, they further take the role of raising the clearing point of liquid crystal compositions.

Content of the first component is 3 to 40 % by weight and preferably 5 to 20 % by weight based on the total weight of liquid crystal composition. When the content is less than 3 % by weight, effect of low Vₜₕ which is an object of the present invention becomes difficult to obtain. Conversely, when the content is increased beyond 40 % by weight, compatibility of liquid crystalline compounds at low temperatures will sometimes be deteriorated, which is not preferable.

Any of the compounds expressed by any one of the formulas (2-1) to (2-7) and compounds of the first group and the second group mentioned above is a trifluoro-compound, and they are well known (R. Tarao et al., SID 94 Digest, p 233) as the compounds for low voltage TFT since they have a Δε in a range of about 7 to about 12 and are excellent in heat stability and chemical stability as clear from the Unexamined Japanese Patent Publication No. 2-233626 mentioned above.

Among them, the compounds of the second component have an upper limit value of T_{NI} in a range of about 50 to 100°C, and thus are most suitable as a base compound of compositions for low voltage TFT.

Its content is 60 to 97 % by weight and preferably 70 to 95 % by weight based on the total weight of liquid crystal composition. When the content is less than 60 % by weight, the compatibility of the liquid crystal composition will sometimes be deteriorated particularly at low temperatures. Conversely, when the content is increased beyond 97 % by weight, low voltage effect which is one of the objects of the present invention becomes difficult to obtain.

Among the first group to the third group compounds which can be further added to the liquid crystal compositions of the present invention, the compounds expressed by the formula (3) of the first group are bicyclic trifluoro-compounds and assume the role of particularly lowering Vₜₕ of liquid crystal composition.

Its content is generally 20 % by weight or less and preferably 15 % by weight or less based on the total weight of liquid crystal composition to prevent the T_{NI} of liquid crystal composition from being excessively lowered.

Next, the compounds expressed by either formula (4-1) or (4-2) of the second group are tetracyclic trifluoro-compounds and assume the role of particularly raising T_{NI} of liquid crystal composition.

However, since they are tetracyclic compounds, they may raise Vₜₕ or deteriorate the compatibility at low temperatures when used in a large amount. Accordingly, its content is 20 % by weight or less and preferably 15 % by weight or less based on the total weight of liquid crystal composition.

Compounds expressed by the formula of (5-1) or (5-2) of the third group are bi- or tricyclic chloride (Cl) type compounds and take the role of principally reducing the viscosity of liquid crystal composition. Since these compounds have a small Δε of 4 to 5, they will sometimes raise Vₜₕ when used in a large amount. Accordingly, its content is 30 % by weight or less and preferably 20 % by weight or less based on the total weight of liquid crystal composition.

Composition of the present invention may comprise another or other compounds in addition to the compounds of the first group to the third group compounds mentioned above with the purpose of the present invention, for example, improving Vₜₕ, compatibility at low temperatures, and nematic phase range.

Liquid crystal compositions of the present invention are produced by conventional methods, for instance, by dissolving several components each other at a high temperature or by dissolving each component in an organic solvent, mixing them, and distilling off the solvent under a reduced pressure.

Further, the compositions are improved by incorporating a suitable additive depending on an intended application and optimized. These additives are well known in the art and described in reference books. Usually, a chiral dopant and other materials are added to induce the spiral structure of liquid crystal composition to adjust a required twist angle and to prevent reverse twist.

Still further, the compositions can be used as the liquid crystal compositions for guest-host (GH) mode if dichronic dye such as a merocyanine type, styryl type, azo type, azomethine type, azoxy type, quinophthalone type, anthraquinone type, and tetrazine type was added. Liquid crystal compositions of the present invention can be used as the ones for a polymer dispersion type liquid crystal display devices (PDLCD) typified by NCAP which is prepared by forming a nematic liquid crystal into a microcapsule or typified by a polymer net work liquid display device (PNLCD) which is prepared by forming a polymer of three-dimensional network structure in a liquid crystal. Also, the liquid crystal compositions of the present invention can be used as ones for electrically controlled birefringence (ECB) type or dynamic scattering (DS) type.

According to the present invention, a liquid crystal composition which particularly has a small V_{th,} is excellent in compatibility at low temperatures, and has a large nematic phase range can be provided with satisfying several characteristics required for liquid crystal compositions for AM-LCD.

### EXAMPLES

The present invention will be described in further detail below with reference to Examples. However, it should be understood that the present invention is by no means restricted by such specific Examples.

In the compositions shown in each of Examples and Comparative Examples, indication of compounds is given according to the definition shown in Table 1 below in which left side end group is expressed by n-, nO-, nOm-, V-, Vn-, nVm-, or nVmVk- (n, m, and k is an integer of 1 or more); bonding group is expressed by 2, 4, E, T, V, CF2O, or OCF2; ring structure is expressed by B, B(F), B(F,F), H, Py, D, or Ch, and right side end group is expressed by -F, -CL, -CF3, -OCF3, -OCF2H, -n, -On, -EMe, -nV, or -mVn (n and m is an integer of 1 or more), respectively.

Data of characteristics of liquid crystal compositions are shown by T_{NI} (clearing point), T_{L} (lower limit value of nematic phase transition temperature), η20 (viscosity at 20°C), Δn (otical anisotropy at 25°C), Δε (dielectric anisotropy at 25°C), Vₜₕ (threshold voltage at 20°C), and VHR (voltage holding ratio obtained based on area method) at 25°C. In these characteristics, T_{L} was judged by the liquid crystal phase after a compositions was left as it is for 30 days in freezers each kept at 0°C, -10°C, -20°C, or -30°C.

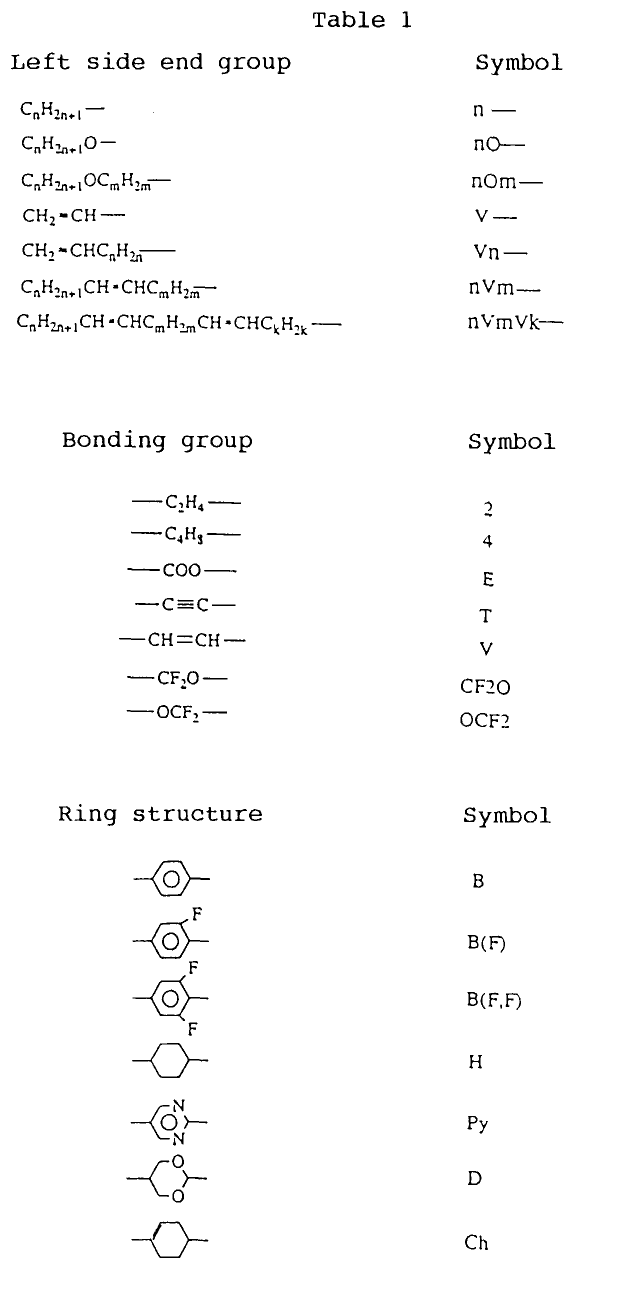

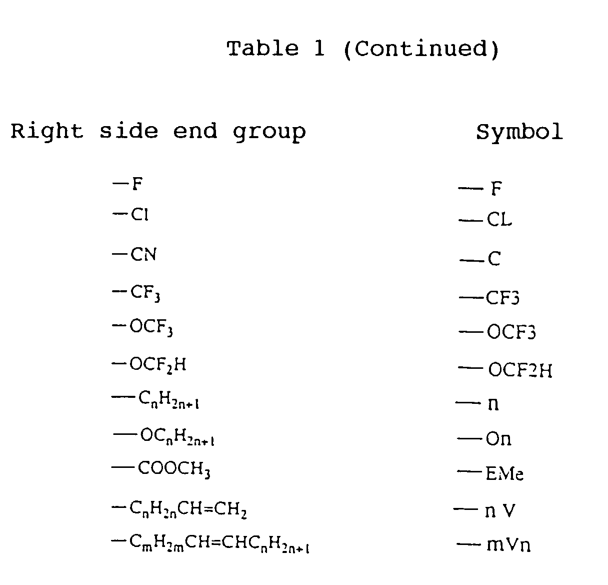

### Comparative Example 1

Following composition is disclosed in Application Example 2 of Unexamined Japanese Patent Publication No. 2-233626 mentioned above:

| | |
|---|---|
| 3 - HHB (F,F) - F | 15.0 % |
| 2 - HHB (F) - F | 28.4 % |
| 3 - HHB (F) - F | 28.3 % |
| 5 - HHB (F) - F | 28.3 % |

Characteristics of this composition were obtained and results were as follows:
T_{NI} = 110.7°C
T_{L} < 0°C
η20 = 25.0 mPa·s
Δn = 0.077
Vₜₕ = 2.32 V
VHR = 98.8 %

As will be clear from the results mentioned above, it can be understood that this liquid crystal composition has a high Vₜₕ and besides the composition is poor in compatibility at low temperatures (T_{L} is high).

### Comparative Example 2

The following composition is disclosed in Example 1 of WO 94/03558:

| | |
|---|---|
| 7 - HB (F,F) - F | 10.0 % |
| 2 - HHB (F,F) - F | 25.0 % |
| 3 - HHB (F,F) - F | 35.0 % |
| 5 - HHB (F,F) - F | 18.0 % |
| 7 - HB (F) - F | 12.0 % |

Characteristics of this composition were obtained and results were as follows:
T_{NI} = 42.9°C
T_{L} < 0°C
η20 = 22.2 mPa.s
Δn = 0.059
Vₜₕ = 1.07 V
VHR = 98.7 % and Δε= 6.1

As will be clear from the results mentioned above, it can be understood that whereas this liquid crystal composition has a low Vₜₕ, it has a low T_{NI} (clearing point), is poor in compatibility at low temperatures (T_{L} is high), and has a small Δn, and thus it is insufficient for practical use.

### Comparative Example 3

The following composition is disclosed in Example 2 of the WO 94/03558 mentioned in the Comparative Example 2:

| | |
|---|---|
| 2 - HHB (F,F) - F | 26.0 % |
| 3 - HHB (F,F) - F | 26.0 % |
| 5 - HHB (F,F) - F | 26.0 % |
| 7 - HB (F) - F | 12.0 % |
| 5 - H2B (F) - F | 10.0 % |

Characteristics of this composition were obtained and results were as follows:
T_{NI} = 46.0°C
T_{L} < 0°C
η20 = 21.6 mPa·s
Δn = 0.058
Vₜₕ = 1.17 V
VHR = 98.5 % and Δε= 6.1

As will be clear from the results mentioned above, it can be understood that whereas this liquid crystal composition has a low Vₜₕ, it has a low T_{NI} (clearing point), is poor in compatibility at low temperatures (T_{L} is high), and has a small Δn, and thus it is insufficient for practical use.

### Comparative Example 4

The following composition is disclosed in Example 4 of the WO 94/03558 mentioned above:

| | |
|---|---|
| 2 - HHB (F,F) - F | 10.0 % |
| 3 - HHB (F,F) - F | 10.0 % |
| 5 - HHB (F,F) - F | 10.0 % |
| 5 - H2B (F) - F | 10.0 % |
| 5 - HEB - F | 7.5 % |
| 7 - HEB - F | 7.5 % |
| 2 - HHB (F) - F | 11.7 % |
| 3 - HHB (F) - F | 11.7 % |
| 5- HHB (F) - F | 11.6 % |
| 3 - HHB - F | 5.0 % |
| 5 - HHEB - F | 2.5 % |
| 7 - HHEB - F | 2.5 % |

Characteristics of this composition were obtained and results were as follows:
T_{NI} = 71.3°C
T_{L} < -20°C
η20 = 19.2 mPa·s
Δn = 0.070
Vₜₕ = 1.77 V
VHR = 98.2 % and Δε = 5.1

As will be clear from the results mentioned above, it can be understood that this liquid crystal composition has a high Vₜₕ for its high clearing point of about 70°C and exhibits a rather small value of Δn.

### Example 1

Liquid crystal composition comprising the following compounds in the following content was prepared:

| | |
|---|---|
| 3O1 - BEB (F) - OCF3 | 5.0 % |
| 3O1 - HBEB (F) - OCF3 | 5.0 % |
| 4O1 - HBEBB (F,F) - CF3 | 10.0 % |
| 3 - H2HB (F,F) - F | 11.0 % |
| 4 - H2HB (F,F) - F | 10.0 % |
| 3 - HHB (F,F) - F | 10.0 % |
| 3 - HH2B (F,F) - F | 15.0 % |
| 5 - HH2B (F,F) - F | 10.0 % |
| 3 - HBB (F,F) - F | 12.0 % |
| 5 - HBB (F,F) - F | 12.0 % |

Characteristics of this composition were obtained and results were as follows:
T_{NI} = 76.9°C
T_{L} < -30°C
η20 = 41.9 mPa·s
Δn = 0.097
Δε = 14.3
Vₜₕ = 1.24 V
VHR = 98.7 %

This liquid crystal composition is excellent in compatibility at low temperatures compared with those in Comparative Examples 1 to 4.

Further, it can be understood that the nematic phase range is large (T_{NI} is high) such an extent that there exits no problem in practical use, Vₜₕ also shows a low value, and the composition is balanced as a whole and sufficient for practical use.

### Example 2

Liquid crystal composition comprising the following compounds in the following content was prepared:

| | |
|---|---|
| 3O1 - BEBB (F,F) - CF3 | 3.0 % |
| 1O1 - HHEBB (F,F) - CF3 | 2.0 % |
| 3 - H2HB (F,F) - F | 5.0 % |
| 5 - H2HB (F,F) - F | 5.0 % |
| 3 - HH2B (F,F) - F | 5.0 % |
| 3 - HBB (F,F) - F | 27.0 % |
| 5 - HBB (F,F) - F | 27.0 % |
| 5 - H2BB (F,F) - F | 3.0 % |
| 3 - HBEB (F,F) - F | 5.0 % |
| 5 - HBEB (F,F) - F | 3.0 % |
| 3 - HHEB (F,F) - F | 10.0 % |
| 5 - HHEB (F,F) - F | 5.0 % |

Characteristics of this composition were obtained and results were as follows:
T_{NI} = 71.6°C
T_{L} < -30°C
η20 = 38.2 mPa·s
Δn = 0.110
Δε = 12.1
Vₜₕ = 1.24 V
VHR = 98.4 %

### Example 3

Liquid crystal composition comprising the following compounds in the following content was prepared:

| | |
|---|---|
| 1O1 - HHEB (F) - OCF3 | 4.0 % |
| 1O1 - HHEB (F,F) - CF3 | 2.0 % |
| 7 - HB (F,F) - F | 7.0 % |
| 3 - H2HB (F,F) - F | 12.0 % |
| 4 - H2HB (F,F) - F | 10.0 % |
| 5 - H2HB (F,F) - F | 10.0 % |
| 3 - HHB (F,F) - F | 10.0 % |
| 3 - HH2B (F,F) - F | 12.0 % |
| 5 - HH2B (F,F) - F | 10.0 % |
| 3 - HBB (F,F) - F | 12.0 % |
| 5 - HBB (F,F) - F | 11.0 % |

Characteristics of this composition were obtained and results were as follows:
T_{NI} = 70.4°C
T_{L} < -30°C
η20 = 27.1 mPa·s
Δn = 0.081
Δε = 8.7
Vₜₕ = 1.51 V
VHR = 98.6 %

### Example 4

Liquid crystal composition comprising the following compounds in the following content was prepared:

| | |
|---|---|
| 4O1 - HBEBB (F,F) - CF3 | 6.0 % |
| 3 - HHB (F,F) - F | 7.0 % |
| 3 - H2HB (F,F) - F | 9.0 % |
| 4 - H2HB (F,F) - F | 9.0 % |
| 3 - HH2B (F,F) - F | 10.0 % |
| 3 - HBB (F,F) - F | 28.0 % |
| 5 - HBB (F,F) - F | 25.0 % |
| 2 - HHBB (F,F) - F | 2.0 % |
| 5 - HHBB (F,F) - F | 4.0 % |

Characteristics of this composition were obtained and results were as follows:
T_{NI} = 82.0°C
T_{L} < -30°C
η20 = 37.7 mPa·s
Δn = 0.112
Δε = 11.3
Vₜₕ = 1.44 V
VHR = 98.5 %

### Example 5

Liquid crystal composition comprising the following compounds in the following content was prepared:

| | |
|---|---|
| 3O1 - BEB (F) - OCF3 | 3.0 % |
| 1O1 - HHEB (F) - OCF3 | 5.0 % |
| 4O1 - HBEBB (F) - CF3 | 5.0 % |
| 5 - HB - CL | 10.0 % |
| 3 - HHB (F,F) - F | 10.0 % |
| 3 - H2HB (F,F) - F | 12.0 % |
| 4 - H2HB (F,F) - F | 10.0 % |
| 5 - H2HB (F,F) - F | 10.0 % |
| 5 - HBB (F,F) - F | 28.0 % |
| 2 - HHB - CL | 4.0 % |
| 5 - HHB - CL | 3.0 % |

Characteristics of this composition were obtained and results were as follows:
T_{NI} = 76.5°C
T_{L} < -30°C
η20 = 31.6 mPa·s
Δn = 0.095
Δε = 9.4
Vₜₕ = 1.55 V
VHR = 98.8 %

### Example 6

Liquid crystal composition comprising the following compounds in the following content was prepared:

| | |
|---|---|
| 1O1 - HHEB (F) - OCF3 | 10.0 % |
| 7 - HB (F,F) - F | 10.0 % |
| 3 - H2HB (F,F) - F | 10.0 % |
| 4 - H2HB (F,F) - F | 10.0 % |
| 5 - H2HB (F,F) - F | 10.0 % |
| 3 - HHB (F,F) - F | 10.0 % |
| 4 - HHB (F,F) - F | 5.0 % |
| 3 - HH2B (F,F) - F | 10.0 % |
| 3 - HBB (F,F) - F | 10.0 % |
| 5 - HBB (F,F) - F | 5.0 % |
| 3 - HHBB (F,F) - F | 5.0 % |
| 3 - HH2BB (F,F) - F | 5.0 % |

Characteristics of this composition were obtained and results were as follows:
T_{NI} = 77.3°C
T_{L} < -30°C
η20 = 30.9 mPa·s
Δn = 0.086
Δε = 9.3
Vₜₕ = 1.53 V
VHR = 98.4 %

### Example 7

Liquid crystal composition comprising the following compounds in the following content was prepared:

| | |
|---|---|
| 3O1 - BEBB (F,F) - CF3 | 2.0 % |
| 2O1 - HBEB (F,F) - OCF3 | 4.0 % |
| 7 - HB - CL | 5.0 % |
| 7 - HB - (F,F) - F | 5.0 % |
| 3 - HHB - (F,F) - F | 10.0 % |
| 4 - HHB - (F,F) - F | 5.0 % |
| 3 - H2HB - (F,F) - F | 10.0 % |
| 4 - H2HB - (F,F) - F | 10.0 % |
| 5 - H2HB - (F,F) - F | 10.0 % |
| 3 - HH2B - (F,F) - F | 5.0 % |
| 3 - HBB - (F,F) - F | 14.0 % |
| 5 - HBB - (F,F) - F | 14.0 % |
| 3 - HHBB (F,F) - F | 6.0 % |

Characteristics of this composition were obtained and results were as follows:
T_{NI} = 74.7°C
T_{L} < -30°C
η20 = 30.0 mPa·s
Δn = 0.105
Δε = 10.4
Vₜₕ = 1.53 V
VHR = 98.6 %

### Example 8

Liquid crystal composition comprising the following compounds in the following content was prepared:

| | |
|---|---|
| 3O1 - HBEB (F,F) - OCF3 | 6.0 % |
| 3O1 - HHEB (F,F) - OCF3 | 6.0 % |
| 7 - HB (F,F) - F | 6.0 % |
| 3 - HBB (F,F) - F | 9.0 % |
| 5 - HBB (F,F) - F | 9.0 % |
| 3 - HHB (F,F) - F | 6.0 % |
| 4 - HHB (F,F) - F | 5.0 % |
| 3 - HH2B (F,F) - F | 10.0 % |
| 5 - HH2B (F,F) - F | 5.0 % |
| 3 - H2HB (F,F) - F | 9.0 % |
| 4 - H2HB (F,F) - F | 8.0 % |
| 5 - H2HB (F,F) - F | 8.0 % |
| 3 - HHEB (F,F) - F | 10.0 % |
| 2 - HBEB (F,F) - F | 3.0 % |

Characteristics of this composition were obtained and results were as follows:
T_{NI} = 74.1°C
T_{L} < -30°C
η20 = 29.1 mPa·s
Δn = 0.084
Δε = 10.4
Vₜₕ = 1.37 V
VHR = 98.4 %

### Example 9

Liquid crystal composition comprising the following compounds in the following content was prepared:

| | |
|---|---|
| 201 - HBEB (F,F) - OCF3 | 9,0 % |
| 7 - HB (F,F) - F | 10.0 % |
| 3 - HBB (F,F) - F | 12.0 % |
| 3 - HH2B (F,F) - F | 9.0 % |
| 5 - HH2B (F,F) - F | 4.0 % |
| 3 - H2HB (F,F) - F | 10.0 % |
| 4 - H2HB (F,F) - F | 10.0 % |
| 5 - H2HB (F,F) - F | 10.0 % |
| 3 - HHEB (F,F) - F | 10.0 % |
| 4 - HHEB (F,F) - F | 3.0 % |
| 5 - HHEB (F,F) - F | 3.0 % |
| 2 - HHBB (F,F) - F | 5.0 % |
| 3 - HHBB (F,F) - F | 5.0 % |

Characteristics of this composition were obtained and results were as follows:
T_{NI} = 82.0°C
T_{L} < -30°C
η20 = 30.0 mPa·s
Δn = 0.086
Δε = 10.7
Vₜₕ = 1.40 V
VHR = 98.5 %

## Claims

1. A liquid crystal composition comprising, as the first component, at least one compound expressed by any one of the following formulas (1-1) to (1-3) 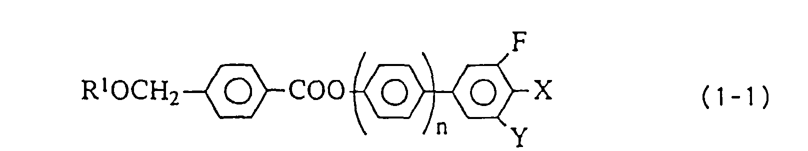 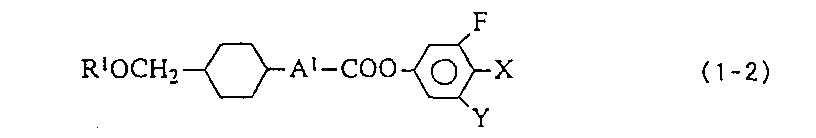 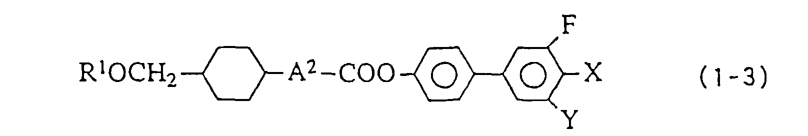 wherein R¹ represents an alkyl group having 1 to 10 carbon atoms, A¹ and A² independently represent trans-1,4-cyclohexylene or 1,4-phenylene, respectively, X represents OCF₃ or CF₃, Y represents H or F, and n represents 0 or 1,
and comprising, as the second component, at least one compound expressed by any one of the following formulas (2-1) to (2-7) 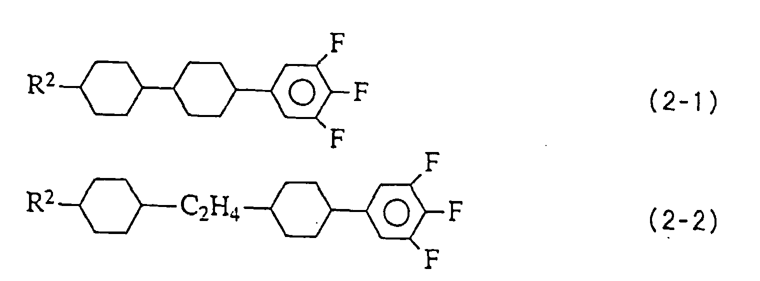 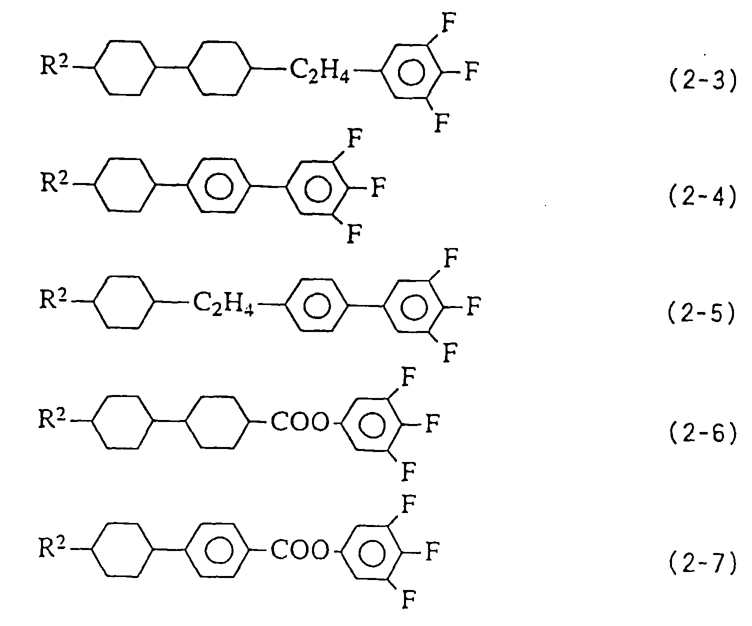 wherein R² represents an alkyl group having 1 to 10 carbon atoms.

2. The liquid crystal composition according to claim 1 wherein the first component and the second component are comprised in the composition in an amount of 3 to 40 % by weight and 60 to 97 % by weight, respectively, based on the total weight of the liquid crystal composition.

3. The liquid crystal composition according to claim 1 or 2 wherein the liquid crystal composition further contains a compound expressed by the following formula (3) 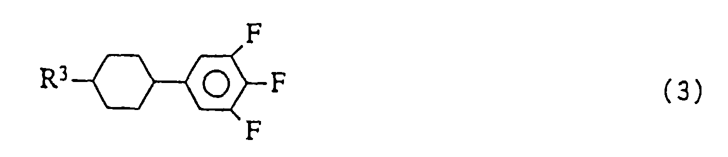 wherein R³ represents an alkyl group having 1 to 10 carbon atoms.

4. The liquid crystal composition according to any one of claims 1 to 3 wherein the liquid crystal composition further comprises a compound expressed by the following formula (4-1) and/or (4-2) 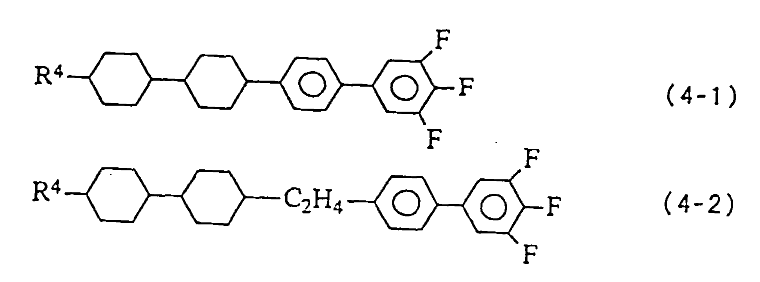 wherein R⁴ represents an alkyl group having 1 to 10 carbon atoms.

5. The liquid crystal composition according to any one of claims 1 to 4 wherein the liquid crystal composition further comprises a compound expressed by the following formula (5-1) and/or (5-2) 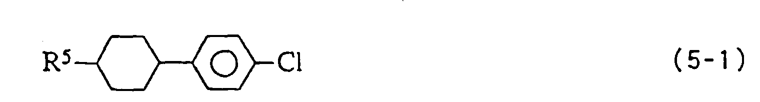 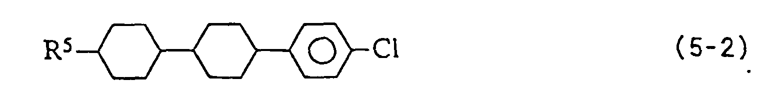 wherein R⁵ represents an alkyl group having 1 to 10 carbon atoms.

6. Liquid crystal display device comprising the liquid crystal composition defined in any one of claims 1-5.

7. Use of the liquid crystal composition defined in any one of claims 1-5 in the production of a liquid crystal display device.

## Patentansprüche

1. Flüssigkristallzusammensetzung, umfassend als erste Komponente mindestens eine Verbindung gemäss einer der folgenden Formeln (1-1) bis (1-3): 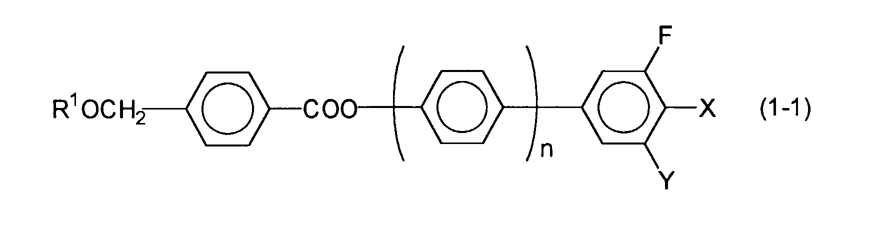 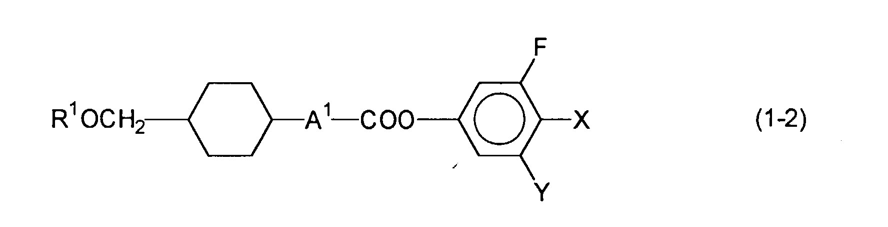 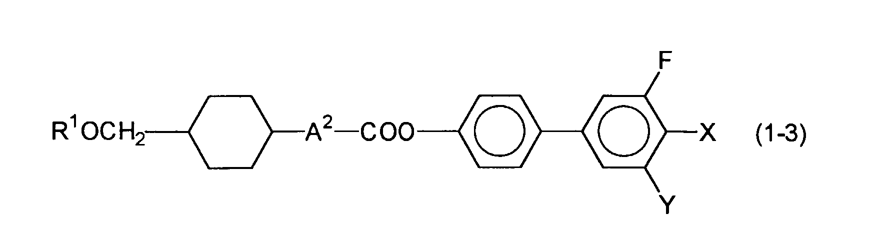 wobei R¹ eine Alkylgruppe mit 1 bis 10 Kohlenstoffatomen darstellt, A¹ und A² jeweils unabhängig trans-1,4-Cyclohexylen oder 1,4-Phenylen darstellen, X OCF₃ oder CF₃ darstellt, Y H oder F darstellt, und n 0 oder 1 darstellt,
und umfassend als zweite Komponente mindestens eine Verbindung gemäss einer der folgenden Formeln (2-1) bis (2-7): 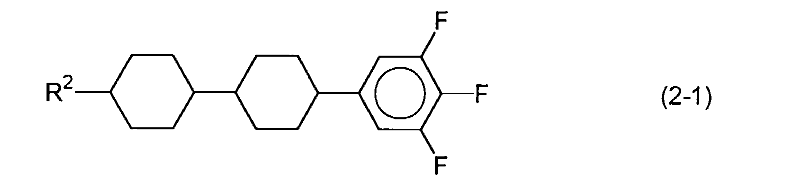 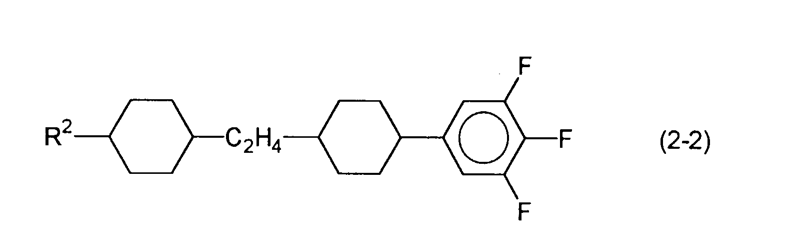 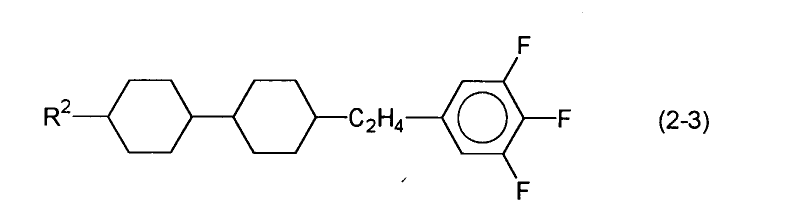 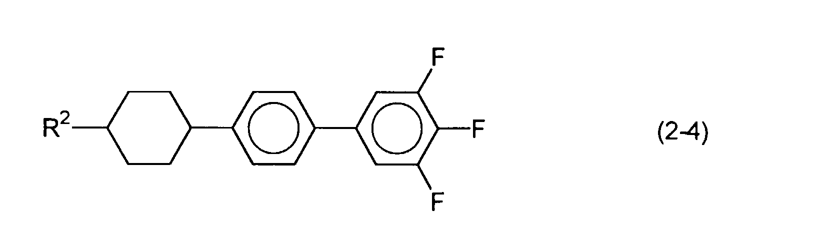 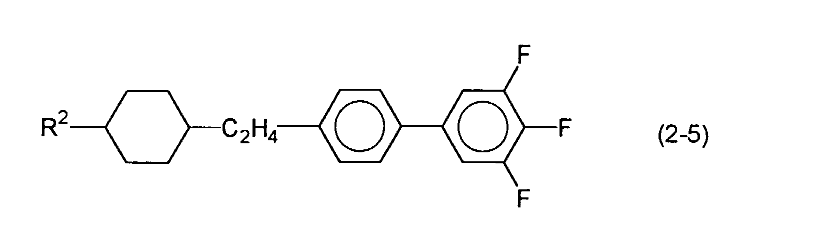 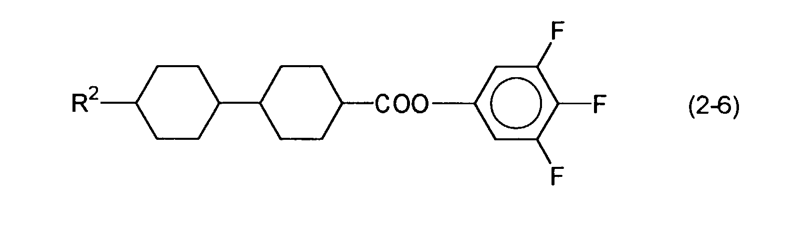 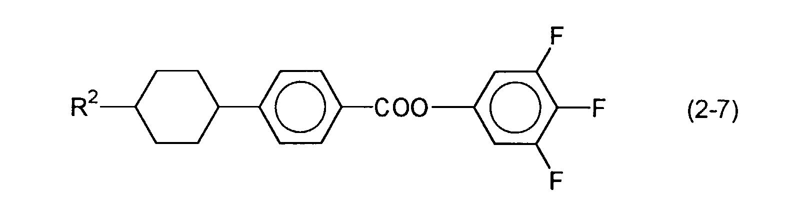 wobei R² eine Alkylgruppe mit 1 bis 10 Kohlenstoffatomen darstellt.

2. Flüssigkristallzusammensetzung gemäss Anspruch 1, wobei die erste Komponente und die zweite Komponente in der Zusammensetzung in einer Menge von jeweils 3 bis 40 Gew.% und 60 bis 97 Gew.% in bezug auf das Gesamtgewicht der Flüssigkristallzusammensetzung enthalten sind.

3. Flüssigkristallzusammensetzung gemäss Anspruch 1 oder 2, wobei die Flüssigkristallzusammensetzung weiterhin eine Verbindung gemäss der folgenden Formel (3) umfasst: 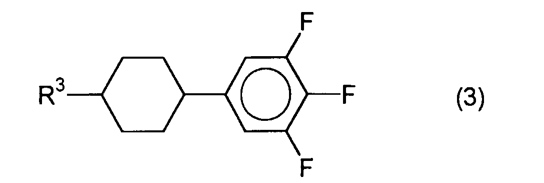 wobei R³ eine Alkylgruppe mit 1 bis 10 Kohlenstoffatomen Druck.

4. Flüssigkristallzusammensetzung gemäss einem der Ansprüche 1 bis 3, wobei die Flüssigkristallzusammensetzung weiterhin eine Verbindung gemäss der folgenden Formel (4-1) und/oder (4-2) umfasst: 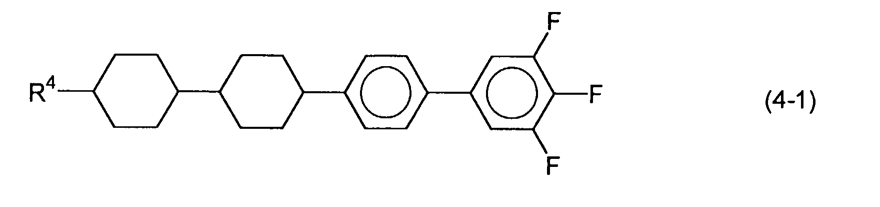 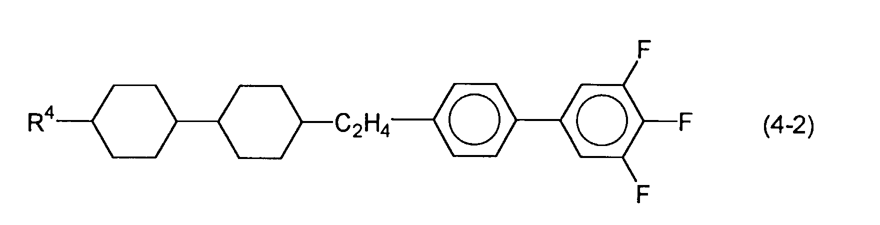 wobei R⁴ eine Alkylgruppe mit 1 bis 10 Kohlenstoffatomen darstellt.

5. Flüssigkristallzusammensetzung gemäss einem der Ansprüche 1 bis 4, wobei die Flüssigkristallzusammensetzung weiterhin eine Verbindung gemäss der folgenden Formel (5-1) und/oder (5-2) umfasst: 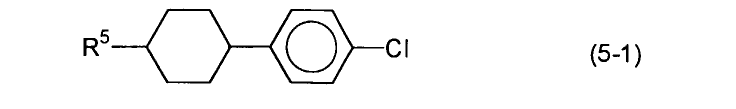 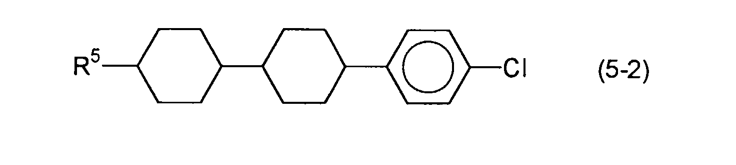 wobei R⁵ eine Alkylgruppe mit 1 bis 10 Kohlenstoffatomen darstellt.

6. Flüssigkristall-Anzeigeeinheit, umfassend die Flüssigkristallzusammensetzung wie in einem der Ansprüche 1 bis 5 definiert.

7. Verwendung der in einem der Ansprüche 1 bis 5 definierten Flüssigkristallzusammensetzungen bei der Herstellung einer Flüssigkristall-Anzeigeeinheit.

## Revendications

1. Composition de cristaux liquides comprenant, comme premier composant, au moins un composé représenté par l'une quelconque des formules (1-1) à (1-3) suivantes : 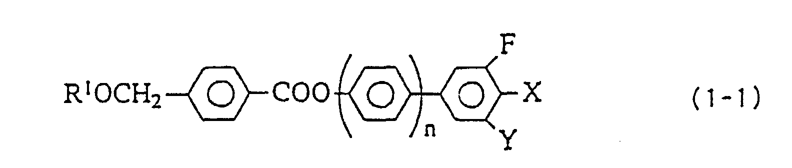 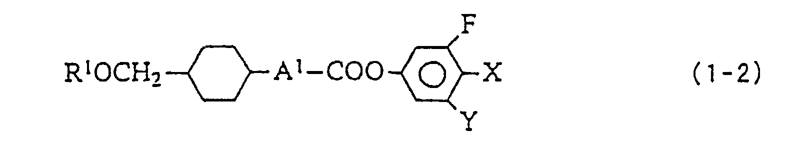 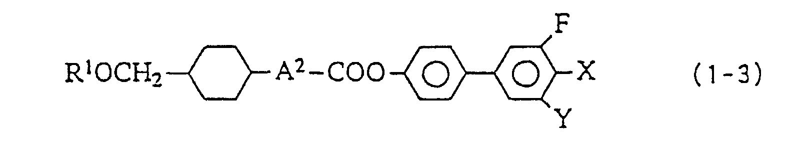 dans lesquelles R¹ représente représente un groupe alkyle comportant 1 à 10 atomes de carbone, A¹ et A² représentent indépendamment respectivement un groupe trans-1,4-cyclohexylène ou 1,4-phénylène, X représente OCF₃ ou CF₃, Y représente H ou F, et n représente 0 ou 1,
et comprenant, comme second composant, au moins un composé représenté par l'une quelconque des formules (2-1) à (2-7) suivantes : 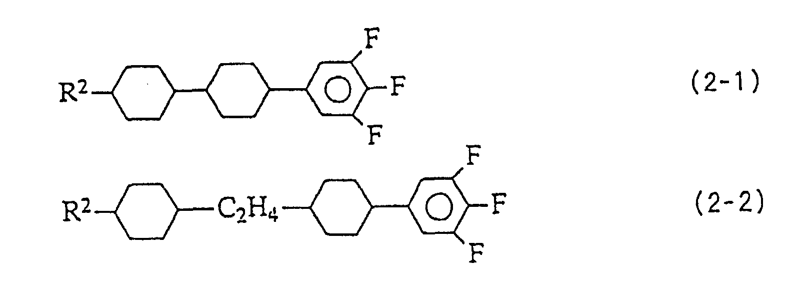 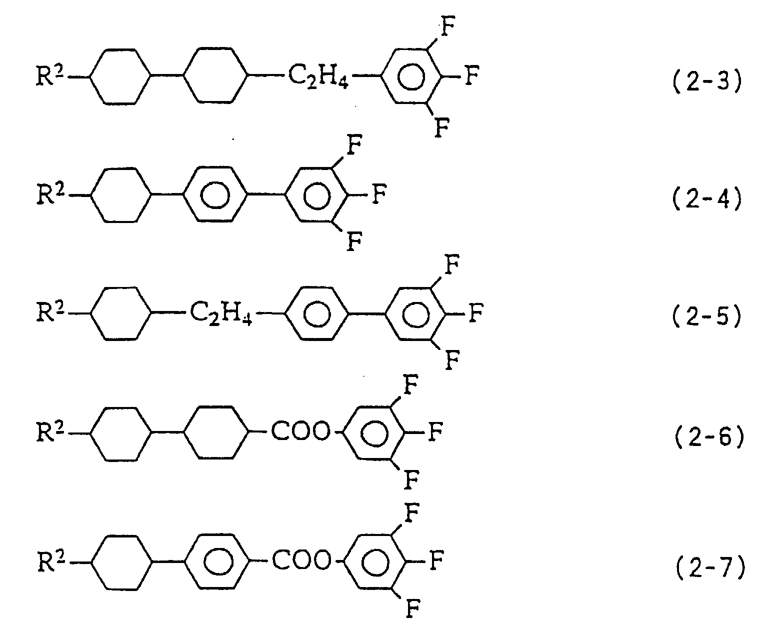 dans lesquelles R² représente un groupe alkyle comportant 1 à 10 atomes de carbone.

2. Composition de cristaux liquides selon la revendication 1, dans laquelle la composition comprend respectivement le premier composant et le second composant en une quantité de 3 à 40 % en poids et de 60 à 97 % en poids, par rapport au poids total de la composition de cristaux liquides.

3. Composition de cristaux liquides selon la revendication 1 ou 2, dans laquelle la composition de cristaux liquides comprend en outre un composé représenté par la formule (3) suivante : 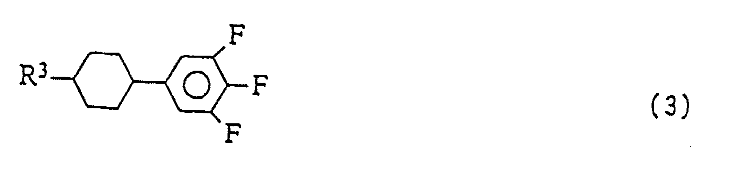 dans laquelle R³ représente un groupe alkyle comportant 1 à 10 atomes de carbone.

4. Composition de cristaux liquides selon l'une quelconque des revendications 1 à 3, dans laquelle la composition de cristaux liquides comprend en outre un composé représenté par les formules (4-1) et/ou (4-2) suivantes : 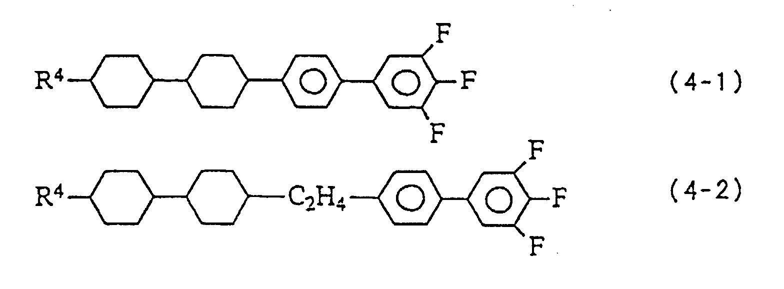 dans lesquelles R⁴ représente un groupe alkyle comportant 1 à 10 atomes de carbone.

5. Composition de cristaux liquides selon l'une quelconque des revendications 1 à 4, dans laquelle la composition de cristaux liquides comprend en outre un composé représenté par les formules (5-1) et/ou (5-2) suivantes : 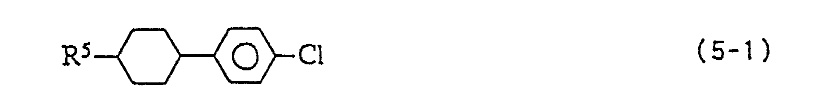 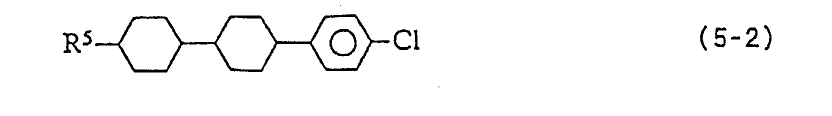 dans lesquelles R⁵ représente un groupe alkyle comportant 1 à 10 atomes de carbone.

6. Dispositif d'affichage à cristaux liquides comprenant la composition de cristaux liquides selon l'une quelconque des revendications 1 à 5.

7. Utilisation de la composition de cristaux liquides selon l'une quelconque des revendications 1 à 5 pour la production d'un dispositif d'affichage à cristaux liquides.
